# EUROPEAN PATENT SPECIFICATION

(11) **EP 3 238 778 B1**
(45) Date of publication and mention of the grant of the patent: **12.06.2019**
(21) Application number: 16166977.5
(22) Date of filing: 26.04.2016
(51) Int. Cl.: A61N 1/375

(54) **HEADER FOR A MEDICAL IMPLANT DEVICE, PARTICULARLY FOR A PACEMAKER**
VORSATZ FÜR EINE MEDIZINISCHE IMPLANTATVORRICHTUNG, INSBESONDERE FÜR EINEN SCHRITTMACHER
COLLECTEUR DESTINÉ À UN DISPOSITIF D'IMPLANT MÉDICAL, EN PARTICULIER POUR UN STIMULATEUR CARDIAQUE

(43) Date of publication of application: 01.11.2017
(73) Proprietor: BIOTRONIK SE & Co. KG, 12359 Berlin (DE)
(72) Inventor: Henschel, Martin, 13125 Berlin (DE); Litzke, Jan, 13507 Berlin (DE)
(74) Representative: Galander, Marcus

(56) References cited:
- EP-A1- 2 529 790
- WO-A1-2011/094413
- US-A1- 2007 232 119
- US-A1- 2010 241 206
- US-A1- 2011 004 124
- US-A1- 2011 137 414
- US-A1- 2013 183 863
- US-A1- 2013 288 501
- US-B1- 8 750 961

## Description

The present invention relates to a header for a medical implant device as well as to a medical implant device comprising such a header.

Such a header (also denoted connection housing) is e.g. used for connecting electrode lines or other (e.g. external) line to a circuit or electrical components of a medical implant device. Such a header usually comprises a header housing which may be made out of an insulating, preferably transparent, material (e.g. which may be a plastic material such as polyurethane (PU), polycarbonate (PC), and/or epoxy resin).

A header of the afore-mentioned kind is for instance disclosed in US 8,676,321.

WO 2011/094413 A1 discloses a battery which comprises a tubular housing, wherein an external surface of the tubular housing comprises a biocompatible material.

US 2013/0288501 A1 describes a connector, an array of connectors, a method for manufacturing the connectors, and a method for using the connectors.

US 2011/0137414 A1 discloses a medical implant which includes a hermetically sealed hollow housing having a hermetically sealed feedthrough suitable for conducting electrical signals to and from the hollow housing without impairing the hermetic seal thereof.

Based on the above, the problem underlying the present invention is to further improve a header of the afore-mentioned kind.

This problem is solved by a header having the features of claim 1 as well as a medical implant device according to claim 11 which uses such a header. Preferred embodiments of these aspects of the present invention are stated in the corresponding sub claims and are described below.

A header for a medical implant device, wherein the header is configured for providing an electrical connection to a circuit of the medical implant device, particularly between at least one electrode and said circuit of the medical implant device, wherein the circuit resides in a housing of the medical implant device, wherein the header further comprises:
- at least one circuit board,
- a header housing which encloses said at least one circuit board and is configured to be connected to said housing of the medical implant device,
   the at least one circuit board is embedded in the housing of the header in a casted material (i.e. a material casted around the printed circuit, particularly by way of injection molding or any other suitable casting process), and
- a sensor system arranged on the at least one circuit board.

Due to using a circuit board that can be pre-mounted with electrical components, particularly sensor systems that are usually arranged in the sealed main housing of the medical implant device a higher integration of the header can be achieved which at the same time allows for an easier automation of the assembly.

According to an embodiment of the header according to the invention, as mentioned initially, the header housing may be transparent and can be made of the materials mentioned above. Further, preferably, the header housing is made of an electrically insulating material and may comprise an externally accessible cavity for connecting an implantable electrode line to the header or other components of the medical implant device that are to be electrically connected to the header.

Particularly, said lines, particularly electrode lines, may be cardiac electrode lines, which may be implanted intracardially or in the form of a coronary sinus (CS) electrode, or nerve or epicardial electrode lines. Such electrode lines may also have another form and function. Apart from electrodes also other components may be connected to the medical implant device by way of the header according to the invention.

Further, according to an embodiment, the circuit board (also denoted as substrate) may be made out of an insulating material, such as a ceramics, liquid crystal polymers (LCP), polyether ether ketones (PEEK), polysulfones (PSU) etc. Advantageously, biocompatible ceramics may be used. Gold, particularly when a ceramics is used as a material for the circuit board, may be used as a material for circuit paths of the circuit board. Particularly, low temperature cofired ceramics (LTCC) are used as (ceramics) material for the at least one circuit board.

According to another embodiment of the invention, the circuit board may comprise one or more layers and may be manufactured and processed using manufacturing methods associated with interconnect devices (MID). Such methods include for instance two (or multi) component injection moulding, hot stamping, laser direct structuring (LDS), mask processing techniques, in-mold film laminating and Flamecon processing. Using the said methods, conductor lines, connectors and contacting components (for instance IS-1/DF-1 connectors, quadrupole connectors, a neurostimulator module and its components as spring sleeve and connector holder, sensors, antenna, X-ray marker, positioning markers for assembling) can be applied, integrated and electrically connected on and in the circuit board.

Further, according to an embodiment of the header according to the present invention, the sensor system is configured to measure at least one of the following quantities: a quantity related to a health status of the patient, blood glucose concentration, blood pressure, blood oxygen concentration, temperature, acceleration, patient posture, respiration, sound, magnetic field, electromagnetic field. The sensor system can further be configured to measure an optical or chemical parameter or a genetic quantity.

According to an embodiment of the header according to the present invention, the sensor system includes sensors located in other parts of the medical implant device, as the device body or the electrode lines, wherein the sensor system performs measurements using one sensor, more than one sensor or a combination of sensors.

Further, according to an embodiment of the header according to the present invention, the header comprises at least one antenna structure for receiving or transmitting signals, which at least one antenna structure is integrated into the at least one circuit board, wherein said at least one antenna structure comprises a plurality of layers integrated into the circuit board. Due to the structure comprising a plurality of stacked layers, the receiving and transmitting capabilities are significantly increased.

Further, according to an embodiment of the header according to the present invention, the at least one circuit board comprises colour and/or symbolic markers for identifying connectors of the header.

Furthermore, according to an embodiment of the header according to the present invention, the at least one circuit board comprises an x-ray marker for generating a defined contrast in an x-ray image of the header.

Further, according to a preferred embodiment of the header according to the present invention, a filter for reducing electromagnetic interference is integrated on the circuit board. For instance, such electromagnetic interference is caused by radiofrequency waves or by the high frequency field of an MRI (magnetic resonance imaging) machine.

Further, according to a preferred embodiment of the header according to the present invention, an energy conversion means for converting energy induced by MRI into photon energy is integrated on the at least one circuit board.

Particularly, such an energy conversion means may comprise a diode array for converting energy induced into the header/medical implant device into photon energy. This allows one to improve MRI compatibility of the medical implant device to a great extent.

Further, according to a preferred embodiment of the header according to the present invention, the at least one circuit board may be a closed circuit board. However, according to a preferred embodiment, the at least one circuit board may comprise at least one recess, particularly a central recess, particularly for the arrangement of intermediary connections members for making connections to electrical components of the header.

Further, the at least one circuit board may comprise one or several optical markers according to an embodiment, particularly for use in an automated mounting and/or welding process.

Further, the at least one circuit board may be obtained by a multi component moulding process, preferably a two component moulding process. For instance, manufacturing and/or further processing of the circuit board involves multi or two component moulding according to processing methods associated with MID.

Further, according to a preferred embodiment of the header according to the present invention, the at least one circuit board comprises contact pads for electrically connecting the at least one circuit board to the medical implant device, particularly to said circuit of the medical implant device (e.g. an electronic control unit of the medical implant device for controlling the latter).

Furthermore, according to an embodiment, the at least one circuit board may comprise structures for fixation of the at least one circuit board in the housing of the header.

Further, according to a preferred embodiment of the header according to the present invention, the header comprises at least one electrically conducting connection assembly being electrically connected to the at least one circuit board and configured for being (e.g. releasably) connected to an electrode (or a plurality of electrodes) or some other component for providing an electrically conducting connection between said electrode(s)/component and the at least one circuit board. Particularly, in an embodiment, said connection assembly comprises one of: a DF-4 connector, an IS-4 connector, a DF-1 connector, an IS-1 connector for making connection to said electrode(s)/component.

Particularly, according to a further embodiment, said connection assembly comprises (particularly apart from one or several contact elements for providing an electrically conducting connection) at least one or several sealing, insulating and/or mounting component(s), particularly out of a thermoplastic or a duroplastic material.

Further, according to an embodiment, the connection assembly may comprise pins being configured to be plugged into corresponding openings of the at least one circuit board (e.g. for making electrical contact and/or for fastening the connection assembly to the at least one circuit board). Further, according to an embodiment, the connection assembly may be configured to be melt on the at least one circuit board for permanently fastening it to the at least one circuit board.

Further according to a preferred embodiment of the present invention, the connection assembly is (e.g permanently) connected to the at least one circuit board via at least one intermediary contact member.

Particularly, the connection assembly may be connected to the at least one intermediary contact member by means of welding, crimping, soldering, plugging, gluing, or by using a corresponding number of spring contacts.

Particularly, in an embodiment, the intermediary contact member is a sheet metal member, particularly a flat sheet metal member or an angled sheet metal member. Other shapes are also conceivable.

Preferably, according to an embodiment of the header according to the present invention, the at least one circuit board is formed as a circumferential frame defining a central recess (see also above), wherein said frame particularly comprises two opposing frame regions, namely a first and an opposing second frame region, which frame regions are integrally connected to each other by two lateral frame regions (namely a third and an opposing fourth frame region), so as to form said frame shape of the at least one circuit board surrounding said central recess of the at least one circuit board.

Further, according to an embodiment, the first and the second frame region are connected to each other by a plurality of intermediary contact members in the form of elongated contact stripes via which the connection assembly is electrically connected to the at least one circuit board.

Particularly, according to an embodiment, said intermediary contact members extend across said central recess of the at least one circuit board. Particularly, in an embodiment said connection assembly is a DF-4 module, i.e., forms an DF-4 connector (see also above), wherein the header then comprises four intermediary contact members in the form of elongated contact stripes that extend across said central recess.

Further, in an embodiment, the header may comprise two further intermediary contact members protruding from the second frame member which is positioned further away from the housing of the medical implant device than the first frame region (when header housing and the housing of the medical implant device are connected to each other), wherein said two further intermediary contact members particularly protrude outwards and away from the central recess/circuit board and are connected to a further connection assembly forming an IS-1 connector.

Further, according to an embodiment, the connection assembly and the further connection assembly are connected to each other, e.g. integrally or may form a pre-mounted assembly. Further, according to an embodiment, the at least one circuit board may comprise a through hole in the second frame member for fixing and/or positioning of said connection assembly (e.g. of the connection assembly and the further connection assembly) to the at least one circuit board.

Further, according to an embodiment, said contact pads for electrically connecting the at least one circuit board to the medical implant device are arranged on the first frame region

According to a further aspect of the present invention, as stated in claim 11, a medical implant device (particularly a pace maker or a bio monitor) is disclosed comprising a housing for accommodating a circuit of the medical implant device (which may comprise a battery, an electronic control unit, e.g. in the form of an integrated circuit, for controlling the medical implant device, and/or one or several electrical components) as well as a header according to the present invention, wherein the header housing is fastened to said housing of the medical implant device.

Particularly, according to an embodiment of medical implant device according to the invention, the housing of the medical implant device comprises a feedthrough via which said at least one circuit board is connected in an electrically conducting fashion to said circuit of the medical implant device, particularly via at least one intermediary contact member. Particularly, due to the feedthrough electrical components (e.g. said circuit) inside the housing of the medical implant device, which housing is preferably hermetically sealed, can be contacted from the outside without breaking said sealing of the housing of the medical implant device.

Further, according to an embodiment of the medical implant device according to the present invention, the at least one intermediary contact member may be connected to the at least one circuit board and/or said feedthrough by means of welding, crimping, soldering, plugging, gluing, or by making a spring contact.

Further, according to an embodiment of the medical implant device according to the present invention, the intermediary contact member is a sheet metal member, particularly a flat sheet metal member, an angled sheet metal member or a sheet metal member forming a sleeve. Other shapes are also conceivable.

Further, according to an embodiment of the medical implant device according to the invention, said feedthrough is a feedthrough assembly comprising a carrier body with a top side and a bottom side; at least one electrical contact body extending on both said top side and said bottom side of the carrier body; wherein the at least one electrical contact body is angled and comprises at least two limbs, and wherein the at least two limbs comprise at least one first limb extending transversely to at least one second limb; an insulating body coupled to the carrier body; wherein the insulating body extends through the carrier body, protrudes over said top side, and projects over said bottom side on said bottom side or is aligned with said bottom side or extends through said carrier body less than a thickness of said carrier body; wherein the at least one electrical contact body is arranged on the insulating body; wherein the carrier body comprises an opening next to said insulating body and at least one positioning unit that locally positions the electrical connecting element; wherein said positioning unit is guided in said opening in a longitudinal direction and extends through said top side and said bottom side of said carrier body; wherein the positioning unit is aligned at least on one end with the at least one electrical contact body; and, wherein the at least one positioning unit comprises a lower end and an upper end, such that the lower end is aligned with a bottom side of said at least one second limb, and the upper end protrudes over said insulating body. Such a feedthrough assembly is also described in detail in US 8,920,198.

Further, according to an embodiment of the medical implant device according to the invention, an integrated circuit is provided on the at least one circuit board of the header, which integrated circuit is configured to control the medical implant device, and wherein said circuit in said housing (particularly merely), which may be a housing out of or comprising Pt, comprises an energy source and/or a capacitor of the medical implant device that may be encapsulated in said housing.

Further, here, the header housing may also be integrally formed with said housing of the medical implant device. Particularly, in this case, the medical implant device may be a so called leadless pacemaker and/or cardiac monitor (i.e. a device for monitoring quantities relating to a health status of the patient) that does not comprise external electrode lines but only internal electrode lines arranged at least in sections in the header.

In the following embodiments of the present invention as well as further features and advantages of the present invention shall be described with reference to the Figures, wherein
- Fig. 1: shows an embodiment of a header of a medical implant device in the form of a pace maker, wherein said header is connected to a housing of the medical implant device;
- Fig. 2: shows the header according to Fig. 1 without its header housing;
- Fig. 3: shows a circuit board of the header;
- Fig. 4: shows a further illustration of the circuit board of Fig. 3;
- Fig. 5: shows the header according to Figs. 1 to 4 connected to a feedthrough assembly of the medical implant device;
- Fig. 6: shows a further embodiment of a header/medical implant device according to the invention, wherein the header is integrally formed with the housing of the medical implant device which optionally merely houses the energy source of the device;
- Fig. 7: shows a close up of an embodiment of a circuit board according to the invention with a multiple layer structure.

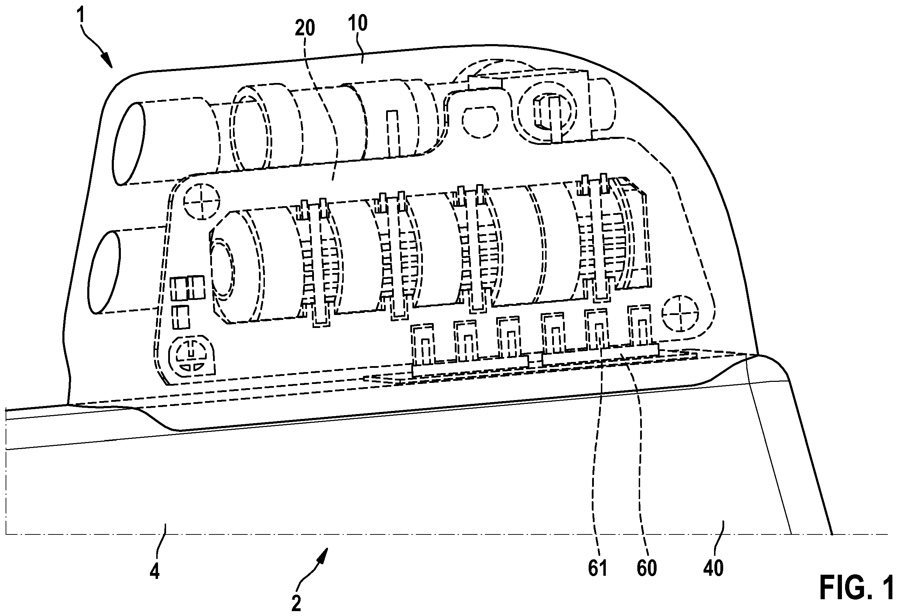

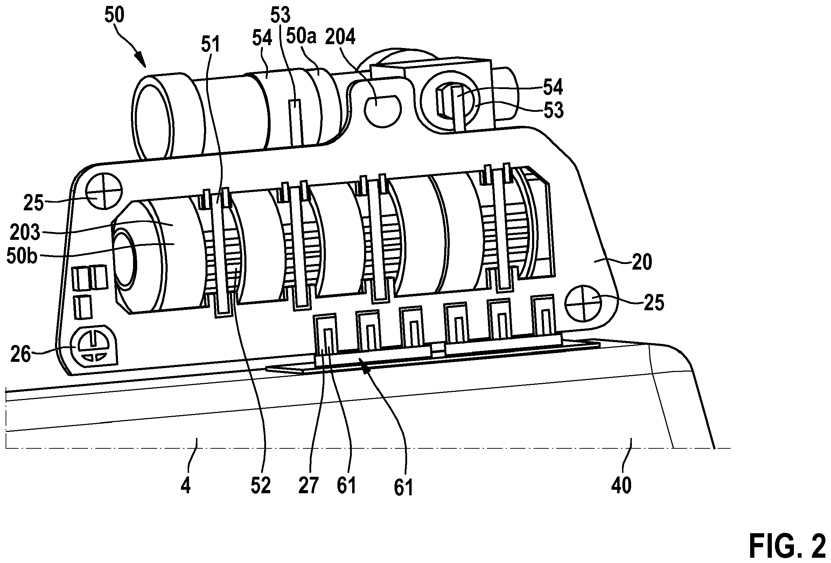

Fig. 1 shows in conjunction with Figs. 2 to 5 a header 1 and medical implant device 2 according to the invention.

The header 1 is configured for providing an electrical connection of at least one electrode 3 to a circuit 40 (not shown in detail) of the medical implant device 2, which circuit 40 resides in a hermetically sealed housing 4 of the medical implant device 2, wherein the header 1 comprises: at least one circuit board 20, a preferably transparent and electrically insulating header housing 10 which encloses said at least one circuit board 20 and is configured to be connected to said housing 4 of the medical implant device 2. According to the invention, a sensor system 30 is provided on the at least one circuit board 20, that is particularly adapted to measure at least one of the following quantities: a quantity related to a health status of the patient, blood glucose concentration, blood pressure, blood oxygen concentration, temperature, acceleration, patient posture, respiration, sound, magnetic field, electromagnetic field.

Preferably, the circuit board 20 is formed out of an LTCC material (see also above).

As can be seen from Figs. 1 to 5, the flat circuit board 20 is preferably formed as a circumferential frame defining a central recess 203, wherein said frame comprises two opposing frame regions, namely a first and an opposing second frame region 21, 22, which frame regions 21, 22 are integrally connected to each other by two lateral frame regions 23, 24 (namely a third and an opposing fourth frame region 23, 24), so as to form said frame shape surrounding said central recess 203 of the frame-shaped circuit board 20.

For connecting two connection assemblies 50a, 50b that may form a pre-mounted or integrally formed connection assembly 50 and that provide e.g. an DF-4 connector and an IS-1 connector for connecting the at least one electrode line, the circuit board 20 comprises a corresponding number of intermediary contact members 51, 53 in the form of elongated metal contact stripes, wherein for making contact to the DF-4 connector four of these intermediary contact members 51 extend from the first frame region 21 to the second frame region 22 across said recess 203 and are welded or soldered to contacts 52 provided on the DF-4 connector 50b. Particularly, said intermediary contact members 51 are attached to first and second frame regions 21, 22 of the circuit board 20 by means of projections 206 which hold the members 51 in place.

Further, for electrically contacting the IS-1 connector 50a, two further intermediary contact members 53 protruding from the second frame member 22 are provided, wherein said two further intermediary contact members 53 protrude outwards away from the central recess 203 and are particularly welded or soldered to corresponding contacts 54 of the IS-1 connector (cf. e.g. Fig. 2).

For fixing and/or positioning of said connector assembly 50 to the circuit board 20, the latter comprises a through hole 204 on the second frame region 22, which particularly comprises a circular boundary section joined by a straight boundary section.

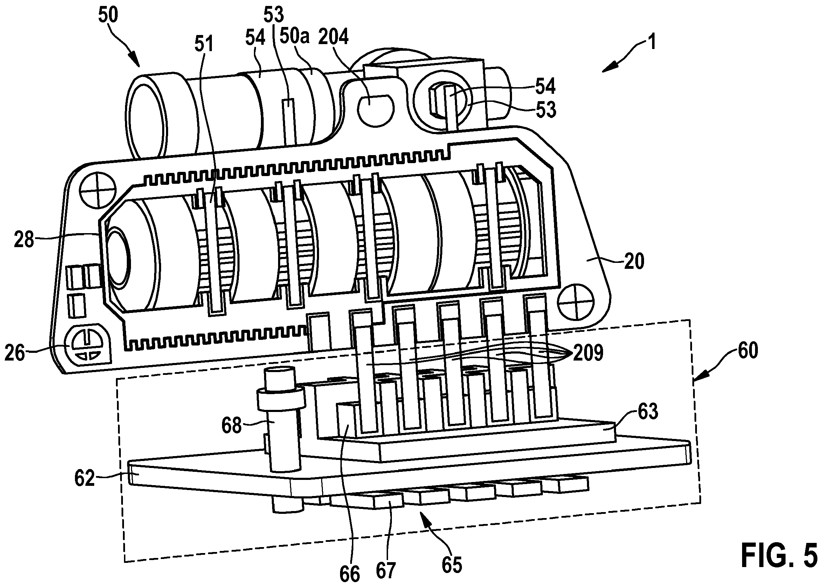

Further, as can be seen from Fig. 5, the circuit board comprises an antenna structure 28 for receiving or transmitting signals extending around the central recess 203 in multiple layers of the circuit board 20, wherein said structure comprises in section a meandering path.

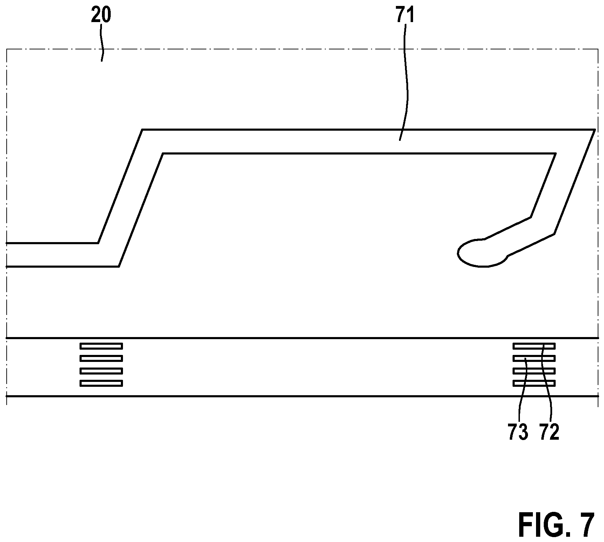

Fig. 7 shows a close up of an embodiment of circuit board 20 according to the invention, having said layer structure comprising three layers 71, 72 and 73.

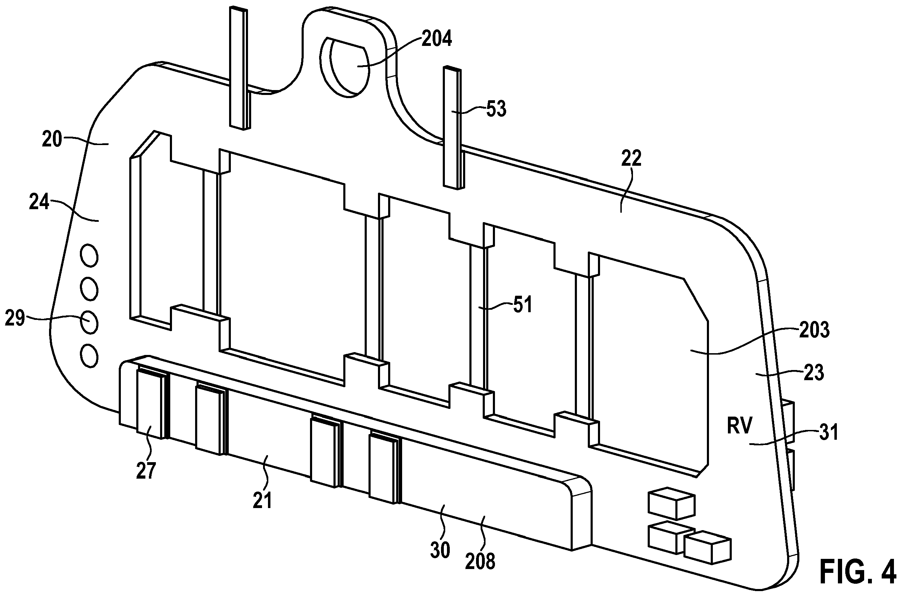

Further, as indicated in Fig. 4 the circuit board comprises colour and/or symbolic markers 29, 31 for identifying the above described connectors 50a, 50b of the header 1. The marker can be implemented as text 31 or as light-emitting diode 29, wherein diodes in different colors can be implemented for indication of different contact states of the connectors 50a, 50b. For example, a red diode glows when an improper contact is detected and a green diode glows, when a proper contact has been established.

Further, as indicated in Figs. 2, 3 and 5, the circuit board preferably comprises an x-ray marker 26 for generating a defined contrast in an x-ray image of the header 1.

Furthermore, the header 1 preferably comprises filter for reducing electromagnetic interference is integrated on the circuit board 20. Further, in the same spirit, for improving MRI compatibility of the medical implant device 2, the header's circuit board 20 preferably also comprises an energy conversion means for converting energy induced by MRI into photon energy. For this, the circuit board 20/energy conversion means may comprise a diode array. The filter and the energy conversion means may be located at any place on the circuit board 20 or implemented as separate components. In one embodiment of the invention, the filter and the energy conversion means are included in the sensor system 30.

Further, preferably, as shown in Figs. 2, 3 and 5 the circuit board 20 preferably comprises optical markers 25 for use in an automated mounting and/or welding process. Here, the markers can be used to determine the position and orientation of the circuit board 20.

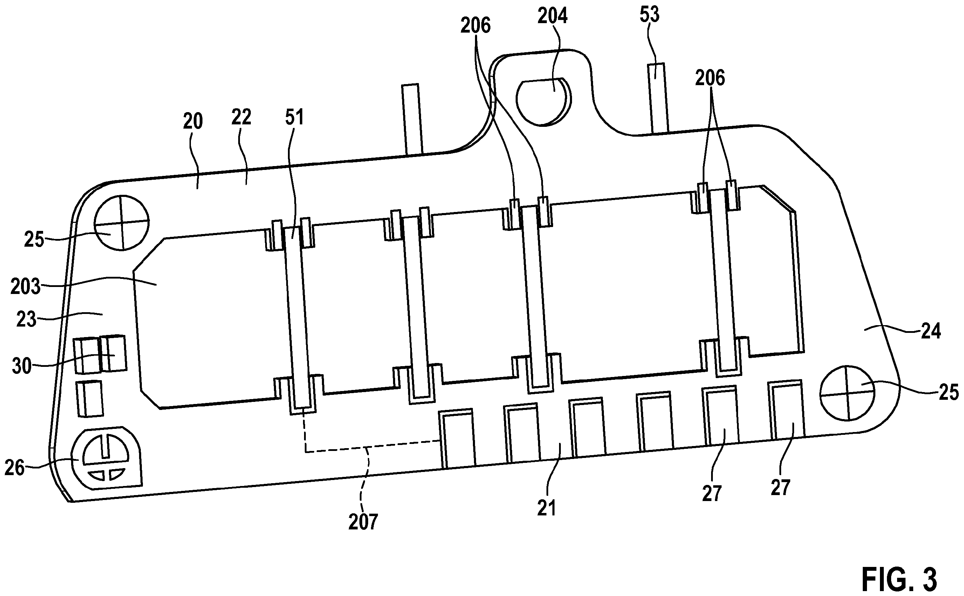

Further, for electrically connecting the circuit board 20 to the circuit 40 of the medical implant device 2, the circuit board 20 preferably comprises contact pads 27 which are preferably arranged on the first frame region 21. Particularly, said contact pads may be connected to the intermediary contact members 51, particularly via an internal multi- or single layer wiring 207 of the circuit board as indicated in Fig. 3.

As shown in the embodiments of Figs. 1 and 2, these contact pads 27 may be welded or soldered to (e.g. straight) pins 61 of a feedthrough 60 of the hermetically sealed housing 4 of the medical implant device 2, which pins 61 protrude out of said housing 2 and are aligned with the contact pads 27.

As an alternative way of connecting the circuit board 20 to the circuit 40 of the medical implant device 2 in housing 4, Fig 5 shows an embodiment that uses a feedthrough assembly 60 being described in detail in US 8,920,198.

According to Fig. 5, the feedthrough assembly 60 comprises a carrier body 62, which bears an insulating body 63. The carrier body 62, which may be connected to a wall of the housing 4 in a sealed fashion, has a top side facing the circuit board 20 and a bottom side facing away from the top side. The insulating body 63 inserted, for example, in an opening of the carrier body 62. It is also conceivable to design the insulating body 63 and carrier body 62 as one piece. The insulating body 63 can advantageously be made of a ceramic material. The use of plastic material or another non-conductive material is also conceivable for the insulating body 63.

Particularly, the insulating body 63 extends through the carrier body 62, protrudes over the top side and also projects slightly over the bottom side on the bottom side. The distance can be used for electrical insulation. However, the insulating body 63 can also be aligned, as needed, with the bottom side, or extend less into the opening of the carrier body 62 than the thickness of the carrier body 62. Further, a positioning unit 68, which is designed as a pin, for example, and is guided in an opening through the carrier body 62, is arranged in a direction normal to the carrier body 62. Further, the insulating body 63 comprises receptacles for receiving contact bodies 65, the receptacles being arranged at regular intervals along the insulating body 63. The contact bodies 65 extend towards the circuit board 20 / contact pads 27 through the carrier body 62 and the insulating body 63. Each contact body 65 comprises a first limb 66 for contacting intermediary contact members (e.g. metal stripes) 209 which are in turn attached to said contact pads 27 on the circuit board, e.g., by means of welding or soldering. Further, each contact body 66 comprises a second limb 67 integrally formed with the associated first limb 66, wherein the second limbs 67 are used to make contact to a circuit 40 (e.g. on a further circuit board) in the housing 4 of the medical implant device 2.

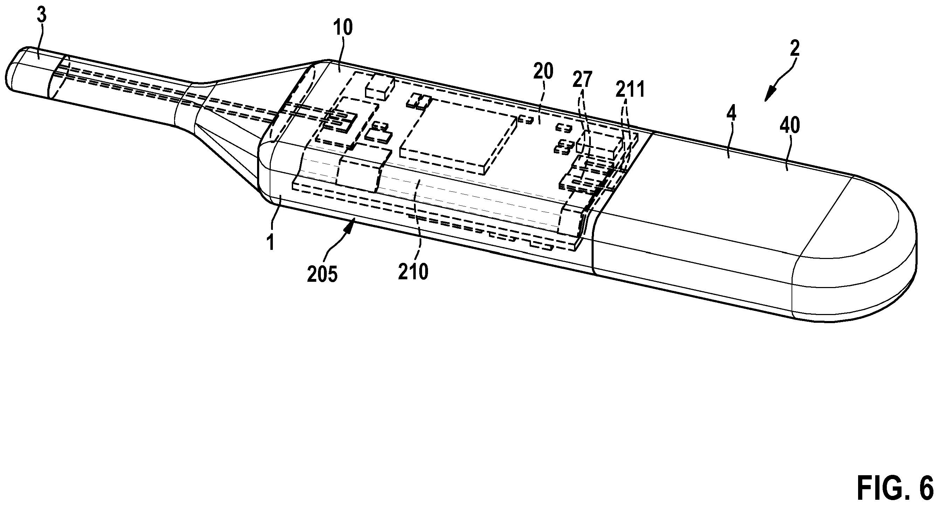

Finally, Fig. 6 shows a further embodiment of the header 1 / medical implant device 2 according to the invention, wherein here an integrated circuit 210 that actually controls the medical implant device 2 is transferred into the header housing 10, which is integrally formed with the housing 4 of the medical implant device which now merely houses a battery 40 for supplying the device 2 with electrical energy. Here, the circuit board 20 of the header may be connected vie contact pads end metal stripes welded thereto to said battery 40. Further, the electrode 3 is integrated into the header housing so that the electrode lines 3a actually are arranged within the header housing 10 which is integrally formed with the (battery) housing 4 of the medical implant device 2. Such a set-up can particularly be used as a so-called leadless pace maker and/or bio monitor. Particularly, the circuit board 20 is embedded in the housing 10 of the header 1 in a casted material 205 being casted around the circuit board 20.

## Claims

1. A header (1) for a medical implant device (2), wherein the header (1) is configured for providing an electrical connection to a circuit (40) of the medical implant device (2), wherein the circuit (40) resides in a housing (4) of the medical implant device (2), wherein the header (1) comprises:
- at least one circuit board (20),
- a header housing (10) which encloses said at least one circuit board (20) and is configured to be connected to said housing (4) of the medical implant device (2), and the at least one circuit board (20) is embedded in the housing of the header in a casted material (205),
**characterized in that**
a sensor system (30) is arranged on the at least one circuit board (20).

2. Header according to claim 1, **characterized in that** the sensor system (30) is configured to measure at least one of the following quantities: a quantity related to a health status of the patient, blood glucose concentration, blood pressure, blood oxygen concentration, temperature, acceleration, patient posture, respiration, sound, magnetic field, electromagnetic field.

3. Header according to claim 1 or 2, **characterized in that** the header (1) comprises at least one antenna structure (28) for receiving or transmitting signals, which at least one antenna structure (28) is integrated into the at least one circuit board (20), wherein said at least one antenna structure (28) comprises a plurality of layers integrated into the at least one circuit board (20).

4. Header according to one of the preceding claims, **characterized in that** the at least one circuit board (20) comprises colour and/or symbolic markers (29, 31) for identifying connectors of the header.

5. Header according to one of the preceding claims, **characterized in that** the at least one circuit board (20) comprises an x-ray marker (26) for generating a defined contrast in an x-ray image of the header (1).

6. Header according to one of the preceding claims, **characterized in that** a filter for reducing electromagnetic interference is integrated on the circuit board.

7. Header according to one of the preceding claims, **characterized in that** an energy conversion means for converting energy induced by MRI into photon energy is integrated on the at least one circuit board (20).

8. Header according to one of the preceding claims, **characterized in that** the at least one circuit board (20) is at least one of: a closed circuit board, a circuit board (20) comprising at least one recess (203), a circuit board (20) comprising an optical marker (25) for use in an automated mounting and/or welding process, a circuit board obtained by a multi component moulding process.

9. Header according to one of the preceding claims, **characterized in that** the header (1) comprises at least one electrically conducting connection assembly (50, 50a, 50b) being electrically connected to the at least one circuit board (20) and configured for being connected to a component of the medical implant device, particularly an electrode, wherein particularly said connection assembly comprises one of: a DF-4 connector (50b), an IS-4 connector, a DF-1 connector, an IS-1 connector (50a).

10. Header according to one of the preceding claims, **characterized in that** the connection assembly is (50) connected to the at least one circuit board (20) via at least one intermediary contact member (51, 53).

11. Medical implant device (2) comprising a housing (4) for accommodating a circuit (40) of the medical implant device (2) as well as a header (1) according to one of the preceding claims, wherein the header housing (10) is connected to said housing (4) of the medical implant device (2).

12. Medical implant device according to claim 11, **characterized in that** the housing (4) of the medical implant device (2) comprises a feedthrough (60) via which said at least one circuit board (20) is connected in an electrically conducting fashion to said circuit (40) of the medical implant device (2), particularly via at least one intermediary contact member (209, 61).

13. Medical implant device according to one of the claim 11 or 12, **characterized in that** an integrated circuit (210) is provided on the circuit board (20) of the header (1), which integrated circuit (210) is configured to control the medical implant device (2), and wherein said circuit (40) in said housing (4) comprises an energy source and/or a capacitor of the medical implant device (2).

14. Medical implant device according to claim 13, **characterized in that** the header housing (10) is integrally formed with said housing (4) of the medical implant device (2).

## Patentansprüche

1. Verbindungskopf (1) für eine medizinische Implantationsvorrichtung (2), wobei der Verbindungskopf (1) zum Bereitstellen einer elektrischen Verbindung mit einer Schaltung (40) der medizinischen Implantationsvorrichtung (2) konfiguriert ist, wobei sich die Schaltung (40) in einem Gehäuse (4) der medizinischen Implantationsvorrichtung (2) befindet, wobei der Verbindungskopf (1) Folgendes aufweist:
- wenigstens eine Leiterplatte (20),
- ein Verbindungskopfgehäuse (10), das die genannte wenigstens eine Leiterplatte (20) umschließt und konfiguriert ist, um mit dem genannten Gehäuse (4) der medizinischen Implantationsvorrichtung (2) verbunden zu werden, und wobei die wenigstens eine Leiterplatte (20) in dem Gehäuse des Verbindungskopfs in einem gegossenen Material (205) eingebettet ist,
**dadurch gekennzeichnet, dass**
ein Sensorsystem (30) auf der wenigstens einen Leiterplatte (20) angeordnet ist.

2. Verbindungskopf nach Anspruch 1, **dadurch gekennzeichnet, dass** das Sensorsystem (30) zum Messen von wenigstens einer der folgenden Größen konfiguriert ist: eine Größe in Bezug auf einen Gesundheitsstatus des Patienten, Blutzuckerkonzentration, Blutdruck, Blutsauerstoffkonzentration, Temperatur, Beschleunigung, Körperhaltung des Patienten, Atmung, Schall, Magnetfeld, elektromagnetisches Feld.

3. Verbindungskopf nach Anspruch 1 oder 2, **dadurch gekennzeichnet, dass** der Verbindungskopf (1) wenigstens eine Antennenstruktur (28) zum Empfangen oder Senden von Signalen aufweist, wobei die wenigstens eine Antennenstruktur (28) in die wenigstens eine Leiterplatte (20) integriert ist, wobei die genannte wenigstens eine Antennenstruktur (28) mehrere Schichten aufweist, die in die wenigstens eine Leiterplatte (20) integriert sind.

4. Verbindungskopf nach einem der vorhergehenden Ansprüche, **dadurch gekennzeichnet, dass** die wenigstens eine Leiterplatte (20) Farb- und/oder symbolische Markierungen (29, 31) zur Kennzeichnung von Verbindern des Verbindungskopfs aufweist.

5. Verbindungskopf nach einem der vorhergehenden Ansprüche, **dadurch gekennzeichnet, dass** die wenigstens eine Leiterplatte (20) einen Röntgenmarker (26) zum Erzeugen eines definierten Kontrasts in einem Röntgenbild des Verbindungskopfs (1) aufweist.

6. Verbindungskopf nach einem der vorhergehenden Ansprüche, **dadurch gekennzeichnet, dass** ein Filter zur Verringerung elektromagnetischer Störung auf der Leiterplatte integriert ist.

7. Verbindungskopf nach einem der vorhergehenden Ansprüche, **dadurch gekennzeichnet, dass** ein Energieumwandlungsmittel zum Umwandeln von durch MRI induzierter Energie in Photonenenergie auf der wenigstens einen Leiterplatte (20) integriert ist.

8. Verbindungskopf nach einem der vorhergehenden Ansprüche, **dadurch gekennzeichnet, dass** die wenigstens eine Leiterplatte (20) wenigstens eine der folgenden ist: eine geschlossene Leiterplatte, eine Leiterplatte (20), die wenigstens eine Aussparung (203) aufweist, eine Leiterplatte (20), die eine optische Markierung (25) zur Verwendung bei einem automatisierten Montage- und/oder Schweißprozess aufweist, eine Leiterplatte, die durch ein Mehrkomponenten-Formverfahren hergestellt wird.

9. Verbindungskopf nach einem der vorhergehenden Ansprüche, **dadurch gekennzeichnet, dass** der Verbindungskopf (1) wenigstens eine elektrisch leitende Verbindungsanordnung (50, 50a, 50b) aufweist, die mit der wenigstens einen Leiterplatte (20) elektrisch verbunden ist und konfiguriert ist, um mit einem Bauteil der medizinischen Implantationsvorrichtung verbunden zu werden, insbesondere einer Elektrode, wobei die genannte Verbindungsanordnung insbesondere einen der folgenden aufweist: einen DF-4-Verbinder (50b), einen IS-4-Verbinder, einen DF-1-Verbinder, einen IS-1-Verbinder (50a).

10. Verbindungskopf nach einem der vorhergehenden Ansprüche, **dadurch gekennzeichnet, dass** die Verbindungsanordnung (50) über wenigstens ein zwischengeschaltetes Kontaktelement (51, 53) mit der wenigstens einen Leiterplatte (20) verbunden ist.

11. Medizinische Implantationsvorrichtung (2), die ein Gehäuse (4) zur Aufnahme einer Schaltung (40) der medizinischen Implantationsvorrichtung (2) sowie einen Verbindungskopf (1) nach einem der vorhergehenden Ansprüche aufweist, wobei das Verbindungskopfgehäuse (10) mit dem genannten Gehäuse (4) der medizinischen Implantationsvorrichtung (2) verbunden ist.

12. Medizinische Implantationsvorrichtung nach Anspruch 11, **dadurch gekennzeichnet, dass** das Gehäuse (4) der medizinischen Implantationsvorrichtung (2) eine Durchführung (60) aufweist, durch welche die genannte wenigstens eine Leiterplatte (20) elektrisch leitend mit der genannten Schaltung (40) der medizinischen Implantationsvorrichtung (2) verbunden ist, insbesondere über wenigstens ein zwischengeschaltetes Kontaktelement (209, 61).

13. Medizinische Implantationsvorrichtung nach Anspruch 11 oder 12, **dadurch gekennzeichnet, dass** auf der Leiterplatte (20) des Verbindungskopfs (1) eine integrierte Schaltung (210) bereitgestellt ist, wobei diese integrierte Schaltung (210) zum Steuern der medizinischen Implantationsvorrichtung (2) konfiguriert ist, und wobei die genannte Schaltung (40) in dem genannten Gehäuse (4) eine Energiequelle und/oder einen Kondensator der medizinischen Implantationsvorrichtung (2) aufweist.

14. Medizinische Implantationsvorrichtung nach Anspruch 13, **dadurch gekennzeichnet, dass** das Verbindungskopfgehäuse (10) mit dem genannten Gehäuse (4) der medizinischen Implantationsvorrichtung (2) einstückig ausgebildet ist.

## Revendications

1. Collecteur (1) pour un dispositif d'implant médical (2), le collecteur (1) étant configuré pour fournir une connexion électrique à un circuit (40) du dispositif d'implant médical (2), où le circuit (40) consiste en un boîtier (4) du dispositif d'implant médical (2), le collecteur (1) comprenant :
- au moins une carte de circuit (20),
- un boîtier de collecteur (10) qui renferme ladite au moins une carte de circuit (20) et est configuré pour être connecté audit boîtier (4) du dispositif d'implant médical (2), et l'au moins une carte de circuit (20) est incorporée dans le boîtier du collecteur dans une matière moulée (205),
**caractérisé en ce**
**qu'**un système de capteur (30) est agencé sur l'au moins une carte de circuit (20).

2. Collecteur selon la revendication 1, **caractérisé en ce que** le système de capteur (30) est configuré pour mesurer au moins l'une des quantités suivantes : une quantité en relation avec un état de santé du patient, une concentration de glucose sanguin, une pression sanguine, une concentration en oxygène dans le sang, une température, une accélération, une posture de patient, une respiration, un son, un champ magnétique, un champ électromagnétique.

3. Collecteur selon la revendication 1 ou la revendication 2, **caractérisé en ce que** le collecteur (1) comprend au moins une structure d'antenne (28) pour recevoir ou transmettre des signaux, laquelle au moins une structure d'antenne (28) est intégrée dans l'au moins une carte de circuit (20), ladite au moins une structure d'antenne (28) comprenant une pluralité de couches intégrées dans l'au moins une carte de circuit (20).

4. Collecteur selon l'une des revendications précédentes, **caractérisé en ce que** l'au moins une carte de circuit (20) comprend des marqueurs de couleurs et/ou de symbolique (29, 31) pour identifier des connecteurs du collecteur.

5. Collecteur selon l'une des revendications précédentes, **caractérisé en ce que** l'au moins une carte de circuit (20) comprend un marqueur de rayons X (26) pour générer un contraste défini dans une image aux rayons X du collecteur (1).

6. Collecteur selon l'une des revendications précédentes, **caractérisé en ce qu'**un filtre pour réduire une interférence électromagnétique est intégré sur la carte de circuit.

7. Collecteur selon l'une des revendications précédentes, **caractérisé en ce qu'**un système de conversion d'énergie pour convertir l'énergie induite par l'IRM en énergie photonique est intégré sur l'au moins une carte de circuit (20).

8. Collecteur selon l'une des revendications précédentes, **caractérisé en ce que** l'au une carte de circuit (20) est au moins une carte parmi : une carte de circuit fermée, une carte de circuit (20) comprenant au moins un renfoncement (203), une carte de circuit (20) comprenant un marqueur optique (25) pour une utilisation dans un procédé de montage automatisé et/ou de soudage, une carte de circuit obtenue par un procédé de moulage de multi composant.

9. Collecteur selon l'une des revendications précédentes, **caractérisé en ce que** le collecteur (1) comprend au moins un ensemble de connexion électriquement conducteur (50, 50a, 50b) étant connecté électriquement à ladite au moins une carte de circuit (20) et configuré pour être connecté à un composant du dispositif d'implant médical, particulièrement une électrode, où, en particulier, ledit ensemble de connexion comprend un connecteur parmi : un connecteur DF-4 (50b), un connecteur IS-4, un connecteur DF-1, un connecteur IS-1 (50a).

10. Collecteur selon l'une des revendications précédentes, **caractérisé en ce que** l'ensemble de connexion (50) est connecté à l'au moins une carte de circuit (20) par le biais d'au moins un élément de contact intermédiaire (51, 53).

11. Dispositif d'implant médical (2) comprenant un boîtier (4) pour loger un circuit (40) du dispositif d'implant médical (2) ainsi qu'un collecteur (1) selon l'une des revendications précédentes, le boîtier de collecteur (10) étant connecté audit boîtier (4) du dispositif d'implant médical (2).

12. Dispositif d'implant médical selon la revendication 11, **caractérisé en ce que** le boîtier (4) du dispositif d'implant médical (2) comprend un passage d'alimentation (60) par le biais duquel ladite au moins une carte de circuit (20) est connectée de manière électriquement conductrice audit circuit (40) du dispositif d'implant médical (2), particulièrement, par le biais d'au moins un élément de contact intermédiaire (209, 61).

13. Dispositif d'implant médical selon la revendication 11 ou la revendication 12, **caractérisé en ce qu'**un circuit intégré (210) est mis en place sur la carte de circuit (20) du collecteur (1), lequel circuit intégré (210) est configuré pour commander le dispositif d'implant médical (2), et où ledit circuit (40) dans ledit boîtier (4) comprend une source d'énergie et/ou un condensateur du dispositif d'implant médical (2).

14. Dispositif d'implant médical selon la revendication 13, **caractérisé en ce que** le boîtier de collecteur (10) est formé intégralement avec ledit boîtier (4) du dispositif d'implant médical (2).
